(19) Europäisches Patentamt
European Patent Office
Office européen des brevets

(11) Publication number: **0 356 091 A2**

# (12) EUROPEAN PATENT APPLICATION

(21) Application number: **89308196.8**

(51) Int. Cl.4: **C07C 201/08 , C07C 205/06**

(22) Date of filing: **11.08.89**

(30) Priority: **19.08.88 GB 8819736**

(43) Date of publication of application:
**28.02.90 Bulletin 90/09**

(84) Designated Contracting States:
**AT BE CH DE ES FR GB GR IT LI LU NL SE**

(71) Applicant: **The British Petroleum Company p.l.c.**
**Britannic House Moor Lane**
**London EC2Y 9BU(GB)**

(72) Inventor: **Butters, Michael**
**Kimben Mongeham Road**
**Ripple Nr. Deal Kent, CT14 8JW(GB)**
Inventor: **Smith, Keith Department of Chemistry**
**University College of Swansea Singleton Park**
**Swansea, SA2 8PP Wales(GB)**
Inventor: **Fry, Karl Department of Chemistry**
**University College of Swansea Singleton Park**
**Swansea, SA2 8PP Wales(GB)**
Inventor: **Stewart, Nevin John**
**The British Petroleum Company p.l.c.**
**Chertsey Road**
**Sunbury-on-Thames Middlesex, TW16 7LN(GB)**

(74) Representative: **Barlow, Michael Thomas et al**
**BP INTERNATIONAL LIMITED Patents & Agreements Division Chertsey Road**
**Sunbury-on-Thames Middlesex TW16 7LN(GB)**

(54) **Nitration of aromatics.**

(57) A process for the para-selective nitration of mono-substituted aromatics comprises reacting the substrate in the liquid phase with an acyl nitrate, e.g. acetyl or benzoyl nitrate and either a zeolite or a clay. The process is particularly suitable for para-nitrating toluene and for nitrating para-terphenyl in para positions on the terminal benzene rings.

EP 0 356 091 A2

# NITRATION OF AROMATICS

The present invention relates to the para-selective nitration of monosubstituted aromatics using a nitrating agent and a zeolite. Nitro-aromatics are widely used in the chemical industry for example in the preparation of dyestuffs.

The nitration of monosubstituted aromatics, e.g. toluene, is an unselective reaction which in general gives rise to a product containing 55-70% of the ortho-nitrated isomer, 30-45% of the para-nitrated isomer and small amounts of the meta. Such a mixture of products is undesirable particularly as it is the para-nitrated isomer which is usually of most value.

The prior art discloses certain procedures for nitrating monosubstituted aromatics which are said to produce greater than 50% of the para-nitrated isomer. For example it has been claimed that low ratios of ortho/para (0.49-0.76) can be obtained by using alkyl nitrates in the presence of polyphosphoric acid. However, such results could not be confirmed by Schofield and co-workers (J. Chem. Soc. Perkin II (1984) p 921).

Another approach, described in J. Chem. Soc. Perkin 11 (1974) p 830, has employed tetranitratotitanium (IV). This procedure has the disadvantage that the nitrating agent is unstable, hence total yields of product vary considerably from experiment to experiment.

Finally, in J. Chem. Soc (Chem. Comm.) (1982) p 1262, a highly selective process (O/P = 1.5-0.25) involving the use of nitronium tetrafluoroborate/crown ether complexes has been disclosed. This process however uses expensive reagents and has not been studied in detail.

The nitration of aromatics in the presence of a range of aluminosilicates, including zeolites, has been described in GB 1560349 and GB 2000141. Both patents describe a heterogeneous gas phase process in which a vapour of the aromatic substrate and nitric acid is passed over the aluminosilicate. Using toluene as the aromatic and montmorillonite as the aluminosilicate, a mixture of isomers of nitrotoluene having the composition ortho 40%; meta 5% and para 55% was produced.

In view of the above prior art, the problem to be solved is to provide a process for nitrating monosubstituted aromatics which is easier to operate and is more para-selective than described previously.

According to the present invention there is provided a process for nitrating a monosubstituted aromatic compound which comprises contacting the monosubstituted aromatic compound with a nitrating agent and an aluminosilicate in the liquid phase characterised in that the nitrating agent is an acyl nitrate and the aluminosilicate is either a zeolite or a clay.

The process of the invention solves the problem defined above by employing an acyl nitrate in combination with a zeolite or clay to effect para-selective nitration under relatively mild conditions. By using an acyl nitrate in combination with a zeolite or clay, a higher selectivity to para-nitrated isomers is obtained than is described in either GB 2000141 or GB 1560349.

The process of the present invention also has the advantages (a) that it avoids the use of the highly corrosive nitrate acid taught in GB 2000141 and GB 1560349 and (b) that it can be operated in the liquid phase.

Considering first the acyl nitrate, this is suitably a compound having the formula $RCONO_3$ wherein R is preferably selected from $C_1$ to $C_6$ alkyl or $C_6$ to $C_{10}$ aryl groups. Most preferred acyl nitrates are acetyl nitrate, benzoyl nitrate or derivatives of benzoyl nitrate in which the benzene ring carries one or more substituents such as $C_1$ to $C_{10}$ alkyl groups, halogen or $C_1$ to $C_{10}$ ether groups.

Whilst any zeolite can in principle be used, the most suitable to date appear to be those having 10 to 12 ring pore openings. Typical examples include mordenite (10 ring) or the synthetic faujasite zeolites zeolite X and Y (12 ring). It is preferable that these zeolites are completely or partially ion-exchanged into a form in which they exhibit acidity. Examples of suitable forms include the hydrogen, aluminium, gallium, lanthanum and ammonium forms. Transition metal forms including the iron, chromium, titanium and vanadium forms can also be used. Heat treated forms, e.g. ultrastable Y, may also be employed. On the basis of the evidence available to date, the partially aluminium exchanged sodium forms appear to be the best.

As an alternative to zeolites, clays may be used. Preferred clays include the smectites such as montmorillonite and like materials. As with the zeolites, it is preferred that the clays are exchanged or otherwise treated to generate forms which exhibit acidity before use.

Whilst the process is believed to be applicable to all monosubstituted aromatic compounds, the process is particularly applicable to monohydrocarbyl substituted aromatic compounds. One important class of such compounds are the monoalkylbenzenes in which the alkyl group is a $C_1$ to $C_{10}$ alkyl group. Another class are compounds of formula:

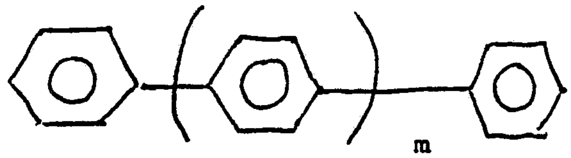

where m is zero or an integer from 1 to 6. Such molecules can be regarded as monohydrocarbyl substituted aromatic compounds in the sense that each of the terminal benzene rings carry one hydrocarbyl radical. In this particular case, it has been observed that both terminal benzene rings can be selectively nitrated at their 4 (i.e. para) positions.

The process of the present invention is suitably carried out in the liquid phase at or near room temperature (i.e. 10-30°C). At high temperatures the process tends to become unselective as the nitrating agent has a tendency to decompose thermally. Lower temperatures can be employed but it appears to make little difference (i.e. no more than 1-2%) to the para selectivity.

The process of the present invention may optionally be carried out in a solvent which is resistant to the nitrating agent, e.g. a halocarbon such as tetrachloromethane, chloroform or dichloromethane.

The process of the present invention can be carried out batchwise in a suitable vessel or continuously by using a trickle bed packed with the zeolite.

The following Examples illustrate the invention.

Examples 1 to 6

A series of glass vessels were charged with 10 ml of tetrachloromethane, 1.5g of mordenite prepared as described below, 2.5 mmol of the appropriate alkylbenzene and 3.25 mmol of benzoyl nitrate (solution in 6.5 ml tetrachloromethane). The contents of each glass vessel was stirred at room temperature for the appropriate time and the products thereafter analysed by gas chromatography.

The results were as follows:

| Example | Alkylbenzene used | Reaction Time (mins) | Yield of Nitrated Product | Selectivity o:m:p |
|---|---|---|---|---|
| 1 | Toluene | 10 | 99 | 32:1:67 |
| 2 | Ethylbenzene | 80 | 100 | 25:2:73 |
| 3 | n-Propylbenzene | 130 | 86 | 26:2:72 |
| 4 | Isopropylbenzene | 70 | 86 | 14:2:84 |
| 5 | Tertbutylbenzene | 70 | 96 | 5:3:92 |
| 6 | n-Hexylbenzene | 100 | 76 | 28:2:70 |

In all cases the para isomer was obtained selectively and in good yield.

Comparative Test A

Example 1 was repeated in the absence of the mordenite. After 24 hours only a 4% yield of nitrated product had been obtained. The ratio of nitrated product was o:m:p = 55:35:10.

Preparation of the Mordenite

20g of the sodium form of large port mordenite were stirred at 100°C for 1 hour in 200 ml of 1 molar ammonium chloride solution. At the end of this time the solution was cooled and the exchanged mordenite recovered by filtration. After water washing and drying at 120°C the mordenite was calcined in air at 550°C. After 18 hours the mordenite was cooled and the whole procedure was repeated this time with 200ml of 1M aluminium chloride solution instead of ammonium chloride solution. After cooling the

mordenite was stored in dry conditions.

Example 7

Para-terphenyl (0.576g, 2.5 mmols) and chloroform (25 mls) were put in a 50 ml round bottom flask together with 1.5g of the mordenite described above. 3.25 mmols of benzoylnitrate (solution in 6.5 ml of chloroform) was then added and the mixture stirred at room temperature for 12 hours. Further benzoyl nitrate (6.5 mmol in 13 ml of carbon tetrachloride) was added and the mixture stirred for a further 12 hours at room temperature. At the end of this time extra chloroform was added, the mordenite removed by filtration, and the crude product separated from the filtrate by evaporation. The crude product was treated with ether to remove impurities and then recrystallised from pyridine. The product (yellow crysals; mp : 276-277° C) was analysed by mass spectroscopy and $^{13}C$ NMR spectroscopy. The results confirmed the product to be 4,4′-dinitroterphenyl, yield 0.57g (71%).

**Claims**

1. A process for nitrating a monosubstituted aromatic compound which comprises contacting the monosubstituted aromatic compound with a nitrating agent and an aluminosilicate in the liquid phase characterised in that the nitrating agent is an acyl nitrate and the aluminosilicate is either a zeolite or a clay.

2. A process as claimed in claim 1 characterised in that the acyl nitrate is selected from acetyl nitrate, benzoyl nitrate or derivatives of benzoyl nitrate in which the benzene rings carry one or more substituents selected from $C_1$ to $C_{10}$ alkyl groups, halogen groups or $C_1$ to $C_{10}$ ether groups.

3. A process as claimed in claim 1 characterised in that the aluminosilicate is a zeolite having 10 to 12 ring pore openings.

4. A process as claimed in claim 3 characterised in that the zeolite is selected from mordenite, zeolite X or zeolite Y.

5. A process as claimed in claim 4 characterised in that the zeolite is completely or partially ion-exchanged into a form in which it exhibits acidity.

6. A process as claimed in claim 5 characterised in that the zeolite is derived from the sodium form of the zeolite by partial aluminium exchange.

7. A process for preparing compounds of the general formula:

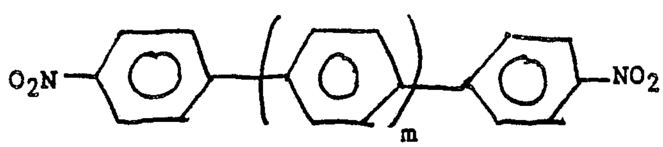

where m is zero or an integer from 1 to 6 characterised by reacting a monosubstituted aromatic compound of formula:

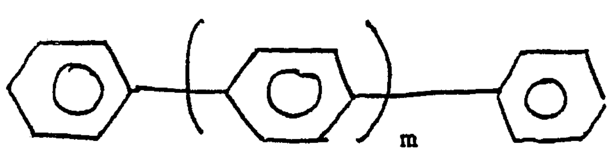

with an acyl nitrate and an aluminosilicate which is either a zeolite or a clay in the liquid phase.